(19) Europäisches Patentamt
European Patent Office
Office européen des brevets

(11) Veröffentlichungsnummer: **0 094 572**
**B1**

(12) # EUROPÄISCHE PATENTSCHRIFT

(45) Veröffentlichungstag der Patentschrift: **12.03.86**

(21) Anmeldenummer: **83104467.2**

(22) Anmeldetag: **06.05.83**

(51) Int. Cl.[4]: **C 07 D 249/08**, C 07 D 233/61, A 01 N 43/04, A 01 N 43/50 // C07C149/34, C07C149/16

(54) **1-Azolyl-2-oximino-butan-Derivate, Verfahren zu ihrer Herstellung sowie ihre Verwendung als Fungizide und Pflanzenwachstumsregulatoren.**

(30) Priorität: **19.05.82 DE 3219041**

(43) Veröffentlichungstag der Anmeldung: **23.11.83 Patentblatt 83/47**

(45) Bekanntmachung des Hinweises auf die Patenterteilung: **12.03.86 Patentblatt 86/11**

(84) Benannte Vertragsstaaten: **AT BE CH DE FR GB IT LI NL SE**

(56) Entgegenhaltungen:
**EP - A - 0 005 249**
**DE - A - 2 613 167**
**DE - A - 2 635 883**
**DE - A - 2 657 578**

(73) Patentinhaber: **BAYER AG, Konzernverwaltung RP Patentabteilung, D-5090 Leverkusen 1 Bayerwerk (DE)**

(72) Erfinder: **Elbe, Hans-Ludwig, Dr., Dasnoeckel 59, D-5600 Wuppertal 11 (DE)**
Erfinder: **Krämer, Wolfgang, Dr., Am Eckbusch 39/45, D-5600 Wuppertal 1 (DE)**
Erfinder: **Büchel, Karl Heinz, Prof., Dr., Dabringhausener Strasse 42, D-5093 Burscheid (DE)**
Erfinder: **Lürssen, Klaus, Dr., August-Kierspel-Strasse 89, D-5060 Bergisch-Gladbach 2 (DE)**
Erfinder: **Reinecke, Paul, Dr., Lessingstrasse 11, D-5090 Leverkusen 3 (DE)**
Erfinder: **Scheinpflug, Hans, Dr., Am Thelenhof 15, D-5090 Leverkusen 1 (DE)**

**EP 0 094 572 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 1-Azolyl-2-oximinobutanderivate, mehrere Verfahren zur ihrer Herstellung sowie ihre Verwendung als Fungizide und Pflanzenwachstumsregulatoren.

Es ist bereits bekannt, dass substituierte 2-Azolyl-1-benzyloximino-1-phenylethane gute fungizide Eigenschaften aufweisen (vgl.: DE-OS 2657578, DE-OS 2723942 und DE-OS 2816817 [Le A 18777]). Die Wirkung dieser Verbindungen ist jedoch, insbesondere bei niedrigen Aufwandmengen und -konzentrationen, nicht immer voll befriedigend.

Es wurden neue 1-Azolyl-2-oximinobutanderivate der allgemeinen Formel (I):

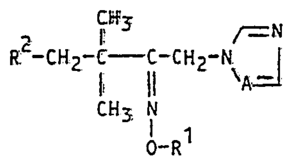
(I)

in welcher

A für Stickstoff oder die CH-Gruppe steht,

$R^1$ für Wasserstoff, für geradkettiges oder verzweigtes Alkyl mit 1 bis 12 Kohlenstoffatomen, für geradkettiges oder verzweigtes Alkenyl oder Alkinyl mit jeweils 2 bis 12 Kohlenstoffatomen, für geradkettiges oder verzweigtes Halogenalkyl mit 1 bis 4 Kohlenstoffatomen und 1 bis 5 gleichen oder verschiedenen Halogenatomen steht, ferner für Cycloalkyl oder Cycloalkenyl mit jeweils 5 bis 7 Kohlenstoffatomen, Cycloalkylalkyl mit 3 bis 7 Kohlenstoffatomen im cycloalkylteil und 1 bis 4 Kohlenstoffatomen im Alkylteil, und schliesslich für gegebenenfalls einfach oder mehrfach, gleich oder verschieden substituiertes Benzyl oder Phenyl steht, wobei als Substituenten Halogen, Cyano, Nitro, Alkyl und Alkoxy mit jeweils 1 bis 4 Kohlenstoffatomen, gegebenenfalls durch Halogen substituiertes Phenyl oder Phenoxy, Halogenalkyl oder Halogenalkoxy mit jeweils 1 bis 3 gleichen oder verschiedenen Halogenatomen zu nennen sind, und

$R^2$ für jeweils gegebenenfalls einfach oder mehrfach, gleich oder verschieden substituiertes Phenyl, Phenoxy, Phenylthio, Phenylsulfinyl oder Phenylsulfonyl steht, wobei als Substituenten Halogen, Nitro, Cyano, ferner Alkyl, Alkoxy oder Alkylthio mit jeweils 1 bis 6 Kohlenstoffatomen, weiterhin Cyclohexyl, sodann Halogenalkyl, Halogenalkoxy und Halogenalkylthio mit jeweils 1 bis 5 gleichen oder verschiedenen Halogenatomen, ausserdem Dialkylamino mit jeweils 1 bis 4 Kohlenstoffatomen in jedem Alkylteil, sodann Alkoxycarbonyl mit 1 bis 4 Kohlenstoffatomen im Alkylteil, schliesslich jeweils gegebenenfalls einfach oder mehrfach durch Halogen und/oder Alkyl mit 1 bis 4 Kohlenstoffatomen substituiertes Phenyl, Phenoxy, Phenylthio, Benzyl, Benzoxy oder Benzylthio zu nennen sind, sowie deren physiologisch verträgliche Säureadditionssalze und Metallsalzkomplexe gefunden.

Die Verbindung der Formel (I) können in der syn- oder anti-Form vorliegen; vorwiegend fallen sie als Gemische beider Formen an.

Weiterhin wurde gefunden, dass man die 1-Azolyl-2-oximinobutanderivate der Formel (I) erhält, wenn man

a) Azolylketone der Formel (II):

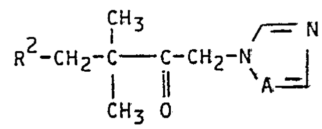
(II)

in welcher A und $R^2$ die oben angegebene Bedeutung haben,

mit substituierten Hydroxylaminen der Formel (III):

$$H_2N-O-R^1 \qquad (III)$$

in welcher $R^1$ die oben angegebene Bedeutung hat,

gegebenenfalls in Gegenwart eines Verdünnungsmittels umsetzt; oder

b) die erfindungsgemässen 1-Azolyl-2-oximinobutanderivate der allgemeinen Formel (Ia):

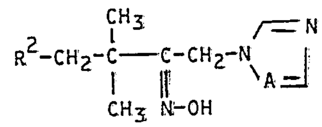
(Ia)

in welcher A und $R^2$ die oben angegebene Bedeutung haben,

mit Halogeniden der Formel (IV):

$$Hal-R^1 \qquad (IV)$$

in welcher

$R^1$ die oben angegebene Bedeutung hat, und

Hal für Chlor oder Brom steht, gegebenenfalls in Gegenwart einer starken Base und in Gegenwart eines Verdünnungsmittels umsetzt, oder

c) 1-Halogen-2-oximinobutanderivate der allgemeinen Formel (V):

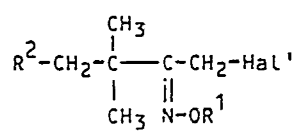
(V)

in welcher

$R^1$ und $R^2$ die oben angegebene Bedeutung haben, und

Hal' für Chlor oder Brom steht, mit 1,2,4-Triazol oder Imidazol in Gegenwart eines Säurebindemittels und in Gegenwart eines Verdünnungsmittels umsetzt.

An die so erhaltenen Verbindungen der Formel (I) kann gegebenenfalls eine Säure oder ein Metallsalz addiert werden.

Schliesslich wurde gefunden, dass die neuen 1-Azolyl-2-oximinobutanderivate der Formel (I) sowie deren Säureadditionssalze und Metallsalzkomplexe starke fungizide und pflanzenwachstumsregulierende Eigenschaften aufweisen.

Überraschenderweise zeigen die erfindungsgemässen Verbindungen der Formel (I) eine bessere

fungizide Wirksamkeit als die aus dem Stand der Technik bekannten substituierten 2-Azolyl-1-benzyloximino-1-phenylethane, welche chemisch und wirkungsmässig naheliegende Verbindungen sind. Zusätzlich zeigen die erfindungsgemässen Verbindungen der Formel (I) überraschend gute pflanzenwachstumsregulierende Wirkung.

Die erfindungsgemässen Stoffe stellen somit eine wertvolle Bereicherung der Technik dar.

Ausserdem sind die erfindungsgemässen Verbindungen der Formel (I), in denen $R^1$ für Wasserstoff steht, interessante Zwischenprodukte. So können z.B. durch Umsetzung mit Acylhalogeniden, Isocyanaten oder Carbamoylchloriden in prinzipiell bekannter Weise Acyl- oder Carbamoylderivate dieser Verbindungen erhalten werden.

Die erfindungsgemässen 1-Azolyl-2-oximino-butanderivate sind durch die Formel (I) allgemein definiert.

Bevorzugt sind diejenigen Verbindungen der Formel (I), in denen

$R^1$ für geradkettiges oder verzweigtes Alkyl mit 1 bis 8 Kohlenstoffatomen, für geradkettiges oder verzweigtes Alkenyl oder Alkinyl mit jeweils 2 bis 8 Kohlenstoffatomen, für Cyclopentyl, Cyclohexyl, Cyclopropylmethyl, Cyclopropylethyl, Cyclopentylmethyl, Cyclohexylmethyl, Cyclohexenyl sowie für gegebenenfalls 1-bis 3fach, gleich oder verschieden substituiertes Benzyl oder Phenyl steht, wobei als Substituenten genannt seien: Fluor, Chlor, Brom, Cyano, Nitro, Methyl, Ethyl, Methoxy, sodann gegebenenfalls durch Fluor oder Chlor substituiertes Phenyl oder Phenoxy sowie Trifluormethyl und Trifluormethoxy, und

$R^2$ für jeweils gegebenenfalls 1- oder 2fach, gleich oder verschieden substituiertes Phenyl, Phenoxy, Phenylthio, Phenylsulfinyl oder Phenylsulfonyl steht, wobei als Substituenten insbesondere genannt seien: Fluor, Chlor, Methyl, gegebenenfalls durch Fluor, Chlor oder Methyl substituiertes Phenyl oder Phenoxy.

Im einzelnen seien ausser den bei den herstellungsbeispielen genannten Verbindungen die folgenden Verbindungen der Formel (I) genannt (A steht sowohl für ein Stickstoffatom als auch für die CH-Gruppe):

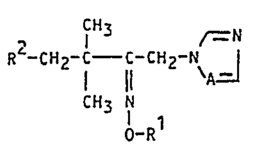
(I)

*(Tabelle auf den nächsten Seiten)*

Verwendet man beispielsweise 4-(4-Chlorphenylsulfenyl)-3,3-dimethyl-1-(imidazol-1-yl)butan-2-on und O-(2-Butyl)hydroxylamin als Ausgangsstoffe, so kann der Reaktionsablauf durch das folgende Formelschema wiedergegeben werden (Verfahren a):

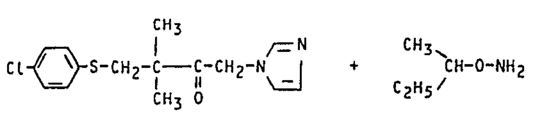

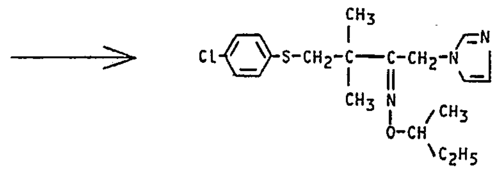

Verwendet man beispielsweise 4-(4-Chlorphenylsulfenyl)-3,3-dimethyl-1-(imidazol-1-yl)-2-oximinobutan und Jodethan als Ausgangsstoffe, so kann der Reaktionsablauf durch das folgende Formelschema wiedergegeben werden (Verfahren b):

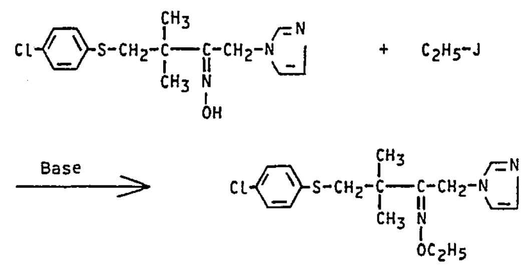


Verwendet man beispielsweise 1-Chlor-4-(4-chlorphenylsulfenyl)-3,3-dimethyl-2-methyloximinobutan und 1,2,4-Triazol als Ausgangsstoffe, so kann der Reaktionsablauf durch das folgende Formelschema wiedergegeben werden (Verfahren c):

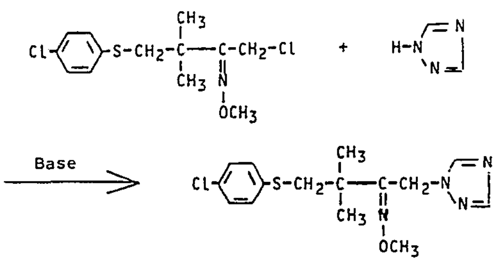


Die bei der Durchführung des erfindungsgemässen Verfahrens (a) als Ausgangsstoffe benötigten Azolylketone sind durch die Formel (II) allgemein definiert. In dieser Formel haben A und $R^2$ vorzugsweise diejenigen Bedeutungen, die bereits im Zusammenhang mit der Beschreibung der erfindungsgemässen Stoffe der Formel (I) vorzugsweise für diese Reste genannt wurden.

Die Azolylketone der Formel (II) sind noch nicht bekannt. Sie sind jedoch Gegenstand einer vorgängigen Deutschen Patentanmeldung (P 3048266 [Le A 20763] vom 20.12.1980). Sie können nach dem dort angegebenen Verfahren erhalten werden, indem man 1-Halogen-2-butanone der Formel (VI):

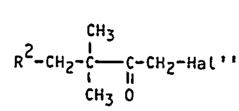
(VI)

in welcher

$R^2$ die oben angegebene Bedeutung hat, und

Hal'' für Chlor oder Brom steht, in üblicher Weise mit 1,2,4-Triazol oder Imidazol in Gegenwart

| R¹ | R² |
|---|---|
| $CH_3$ | 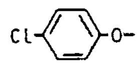 |
| $CH_3$ | 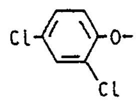 |
| $CH_3$ | 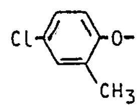 |
| $CH_3$ | 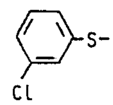 |
| $CH_3$ | 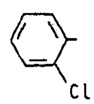 |
| $CH_3$ | 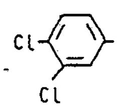 |
| $CH_3$ | 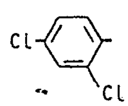 |
| $CH_3$ | 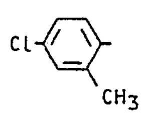 |
| $CH_3$ | 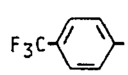 |
| $C_2H_5$ | 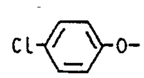 |
| $C_2H_5$ | 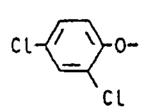 |
| $C_2H_5$ | 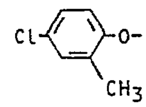 |
| $C_2H_5$ | 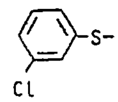 |
| $C_2H_5$ | 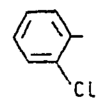 |
| $C_2H_5$ | 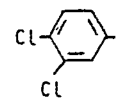 |
| $C_2H_5$ | 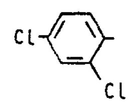 |
| $C_2H_5$ | 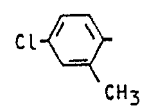 |
| $C_2H_5$ | 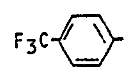 |
| n-$C_3H_7$ | 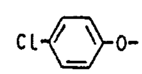 |
| n-$C_3H_7$ | 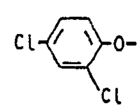 |
| n-$C_3H_7$ | 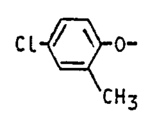 |
| n-$C_3H_7$ | 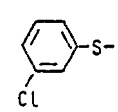 |

| R¹ | R² |
|---|---|
| n-$C_3H_7$ | 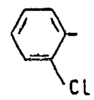 |
| n-$C_3H_7$ | 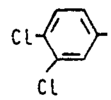 |
| n-$C_3H_7$ | 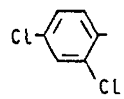 |
| n-$C_3H_7$ | 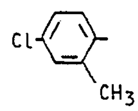 |
| n-$C_3H_7$ | 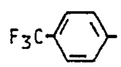 |
| i-$C_3H_7$ | 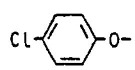 |
| i-$C_3H_7$ | 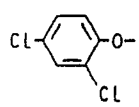 |
| i-$C_3H_7$ | 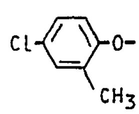 |
| i-$C_3H_7$ | 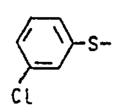 |
| i-$C_3H_7$ | 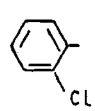 |
| i-$C_3H_7$ | 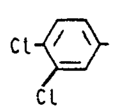 |

| R¹ | R² |
|---|---|
| i-$C_3H_7$ | 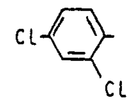 |
| i-$C_3H_7$ | 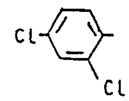 |
| i-$C_3H_7$ | 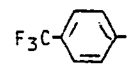 |
| n-$C_4H_9$ | 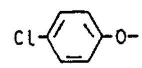 |
| n-$C_4H_9$ | 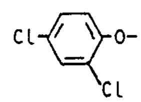 |
| n-$C_4H_9$ | 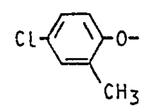 |
| n-$C_4H_9$ | 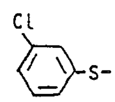 |
| n-$C_4H_9$ | 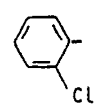 |
| n-$C_4H_9$ | 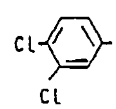 |
| n-$C_4H_9$ | 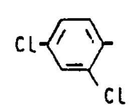 |
| n-$C_4H_9$ | 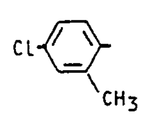 |

| $R^1$ | $R^2$ |
|---|---|
| n-$C_4H_9$ | 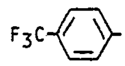 |
| i-$C_4H_9$ | 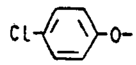 |
| i-$C_4H_9$ | 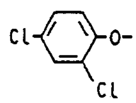 |
| i-$C_4H_9$ | 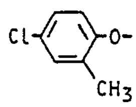 |
| i-$C_4H_9$ | 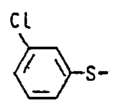 |
| i-$C_4H_9$ | 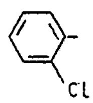 |
| i-$C_4H_9$ | 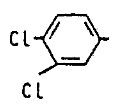 |
| i-$C_4H_9$ | 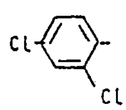 |
| i-$C_4H_9$ | 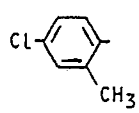 |
| i-$C_4H_9$ | 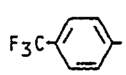 |
| $CH_2=CH-CH_2-$ | 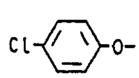 |
| $CH_2=CH-CH_2-$ | 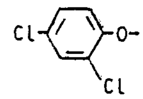 |
| $CH_2=CH-CH_2-$ | 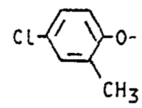 |
| $CH_2=CH-CH_2-$ | 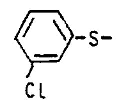 |
| $CH_2=CH-CH_2-$ | 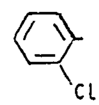 |
| $CH_2=CH-CH_2-$ | 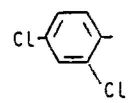 |
| $CH_2=CH-CH_2-$ | 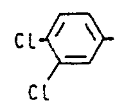 |
| $CH_2=CH-CH_2-$ | 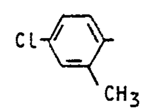 |
| $CH_2=CH-CH_2-$ | 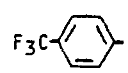 |
| $CH\equiv C-CH_2-$ | 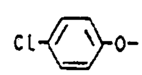 |
| $CH\equiv C-CH_2-$ | 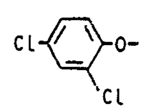 |
| $CH\equiv C-CH_2-$ | 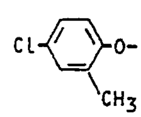 |

| $R^1$ | $R^2$ |
|---|---|
| $CH \equiv C-CH_2-$ | 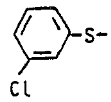 |
| $CH \equiv C-CH_2-$ | 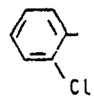 |
| $CH \equiv C-CH_2-$ | 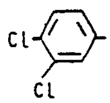 |
| $CH \equiv C-CH_2-$ | 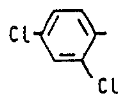 |
| $CH \equiv C-CH_2-$ | 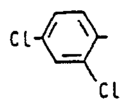 |
| $CH \equiv C-CH_2-$ | 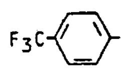 |
| 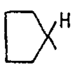 | 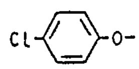 |
| 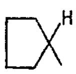 | 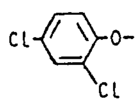 |
| 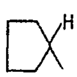 | 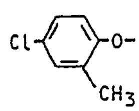 |
| 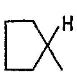 | 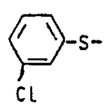 |
| 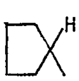 | 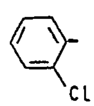 |
| 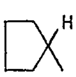 | 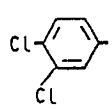 |

| $R^1$ | $R^2$ |
|---|---|
| 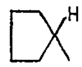 | 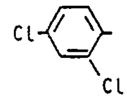 |
| 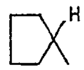 | 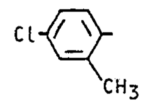 |
| 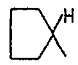 | 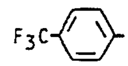 |
| $CH_3-CH=CH-CH_2-$ | 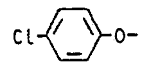 |
| $CH_3-CH=CH-CH_2-$ | 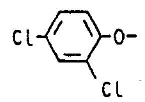 |
| $CH_3-CH=CH-CH_2-$ | 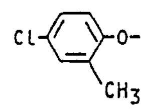 |
| $CH_3-CH=CH-CH_2-$ | 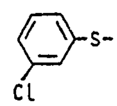 |
| $CH_3-CH=CH-CH_2-$ | 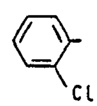 |
| $CH_3-CH=CH-CH_2-$ | 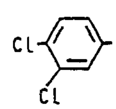 |
| $CH_3-CH=CH-CH_2-$ | 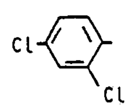 |
| $CH_3-CH=CH-CH_2-$ | 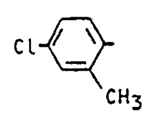 |
| $CH_3-CH=CH-CH_2-$ | 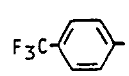 |

eines inerten, organischen Lösungsmittels, wie z.B. Dimethylformamid und in Gegenwart eines Säurebindemittels, wie z.B. Kaliumcarbonat bei Temperaturen zwischen 40 und 120°C umsetzt.

Die ausserdem für das erfindungsgemässe Verfahren (a) als Ausgangsstoffe benötigten gegebenenfalls substituierten Hydroxylamine sind durch die Formel (III) allgemein definiert. In dieser Formel steht $R^1$ vorzugsweise für diejenigen Reste, die bereits im Zusammenhang mit der Beschreibung der erfindungsgemässen Stoffe der Formel (I) vorzugsweise für diesen Substituenten genannt wurden.

Die gegebenenfalls substituierten Hydroxylamine der Formel (III) sind allgemein bekannte Verbindungen der organischen Chemie.

Die bei der Durchführung des erfindungsgemässen Verfahrens (b) als Ausgangsstoffe benötigten 1-Azolyl-2-oximinobutanderivate sind durch die Formel (Ia) allgemein definiert. In dieser Formel haben $R^2$ und A diejenigen Bedeutungen, die bereits im Zusammenhang mit der Beschreibung der erfindungsgemässen Stoffe der Formel (I) vorzugsweise für diese Reste genannt wurden. Die Verbindungen der Formel (Ia) sind erfindungsgemässe Stoffe und werden nach dem erfindungsgemässen Verfahren (a) hergestellt.

Die ausserdem für das erfindungsgemässe Verfahren (b) als Ausgangsstoffe benötigten Halogenide sind durch die Formel (IV) allgemein definiert. In dieser Formel steht $R^1$ vorzugsweise für diejenigen Reste, die bereits im Zusammenhang mit der Beschreibung der erfindungsgemässen Stoffe der Formel (I) vorzugsweise für diesen Substituenten genannt wurden. Hal steht vorzugsweise für Chlor oder Brom.

Die Halogenide der Formel (IV) sind allgemein bekannte Verbindungen der organischen Chemie.

Die bei der Durchführung des erfindungsgemässen Verfahrens (c) als Ausgangsstoffe benötigten 1-Halogen-2-oximinobutanderivate sind durch die Formel (V) allgemein definiert. In dieser Formel stehen $R^1$ und $R^2$ vorzugsweise für diejenigen Reste, die bereits im Zusammenhang mit der Beschreibung der erfindungsgemässen Stoffe der Formel (I) vorzugsweise für diese Substituenten genannt wurden.

Die 1-Halogen-2-oximinobutanderivate der Formel (V) sind noch nicht bekannt. Sie können jedoch in allgemein bekannter Art und Weise erhalten werden, indem man 1-Halogen-2-butanone der Formel (VI) mit gegebenenfalls substituierten Hydroxylaminen der Formel (III) in Gegenwart eines Lösungsmittels, vorzugsweise eines Alkohols, bei 50 bis 100°C umsetzt, wobei die gegebenenfalls substituierten Hydroxylamine vorzugsweise als Hydrochloride in Gegenwart eines Säurebinders eingesetzt werden.

Als Verdünnungsmittel kommen für das erfindungsgemässe Verfahren (a) vorzugsweise Alkohole und Wasser bzw. Gemische beider in Frage.

Die Reaktionstemperaturen können beim Verfahren (a) in einem grösseren Bereich variiert werden. Im allgemeinen arbeitet man zwischen 20 und 120, vorzugsweise zwischen 50 und 100°C.

Bei der Durchführung des erfindungsgemässen Verfahrens (a) setzt man auf 1 Mol Azolylketon der Formel (II) vorzugsweise 1 bis 1,3 Mol Hydroxylamin der Formel (III) ein. Die Isolierung der Verbindungen der Formel (I) erfolgt nach üblichen Methoden.

Nach einer bevorzugten Ausführungsform des Verfahrens (a) werden die Hydroxylamine der Formel (III) in Form ihrer Salze, insbesondere als Hydrochloride, gegebenenfalls in Gegenwart eines Säurebindemittels, wie beispielsweise Natriumacetat, eingesetzt (vgl. auch die Herstellungsbeispiele).

Für die erfindungsgemässe Umsetzung gemäss Verfahren (b) kommen als Verdünnungsmittel inerte organische Lösungsmittel in Frage. Hierzu gehören vorzugsweise Ether, wie Tetrahydrofuran und Dioxan, aromatische Kohlenwasserstoffe, wie Toluol und Benzol, in einzelnen Fällen auch chlorierte Kohlenwasserstoffe, wie Chloroform, Methylenchlorid oder Tetrachlorkohlenstoff, sowie Hexamethylphosphorsäuretriamid, Säureamide, wie Dimethylformamid und Sulfoxide, wie Dimethylsulfoxid.

Die erfindungsgemässe Umsetzung gemäss Verfahren (b) wird gegebenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quaternäre Ammoniumhydroxide und Phosphoniumhydroxide, wie beispielsweise Tetramethylammoniumhydroxid, Benzyltrimethylammoniumhydroxid oder Dibenzyldimethylammoniumhydroxid und Tetraphenylphosphoniumhydroxid oder Methyltriphenylphosphoniumhydroxid.

Die Reaktionstemperaturen können beim Verfahren (b) in einem grösseren Bereich variiert werden. Im allgemeinen arbeitet man zwischen 20 und 150°C, vorzugsweise bei Raumtemperatur. In einzelnen Fällen ist es vorteilhaft, bei der Siedetemperatur des Lösungsmittels, beispielsweise zwischen 60 und 100°C, zu arbeiten.

Bei der Durchführung des erfindungsgemässen Verfahrens (b) setzt man auf 1 Mol Oxim der Formel (Ia) vorzugsweise 1 bis 3 Mol Halogenid der Formel (IV) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform des Verfahrens (b) wird die erfindungsgemässe Umsetzung in einem Zweiphasensystem, wie beispielsweise wässerige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an der Grenzfläche die Ethylate entstehen und mit den in der organischen Phase befindlichen Halogeniden umgesetzt werden.

Für die erfindungsgemässe Umsetzung gemäss Verfahren (c) kommen als Verdünnungsmittel vorzugsweise inerte organische Lösungsmittel in Frage. Hierzu gehören Nitrile, wie Acetonitril, Alkohole, wie Ethanol; Ether, wie Tetrahydrofuran oder Dioxan, aromatische Kohlenwasserstoffe, wie Toluol und Benzol, Formamide, wie Dimethylformamid; sowie halogenierte Kohlenwasserstoffe, wie Methylenchlorid, Tetrachlorkohlenstoff oder Chloroform.

Die erfindungsgemässe Umsetzung gemäss Verfahren (c) wird in Gegenwart eines Säurebindemittels vorgenommen. Man kann alle üblicherweise verwendbaren anorganischen oder organischen Säurebinder zugeben, wie Alkalicarbonate, z.B. Natriumcarbonat und Kaliumcarbonat oder wie niedere tertiäre Alkylamine, Cycloalkylamine oder Aralkylamine, z.B. Triethylamin, N,N-Dimethylcyclohexylamin und N,N-Dimethylbenzylamin. Ebenfalls möglich ist ein entsprechender Überschuss an 1,2,4-Triazol bzw. Imidazol.

Die Reaktionstemperaturen können beim Verfahren (c) in einem grösseren Bereich variiert werden. Im allgemeinen arbeitet man zwischen 0 und 150, vorzugsweise zwischen 60 und 120°C.

Zur Durchführung des erfindungsgemässen Verfahrens (c) setzt man auf 1 Mol der Verbindungen der Formel (V) vorzugsweise 1 bis 2 Mol 1,2,4-Triazol bzw. Imidazol und 1 bis 2 Mol Säurebinder ein. Die Isolierung der Verbindungen der Formel (I) erfolgt nach üblichen Methoden.

Gemäss einer besonderen Ausführungsform des Verfahrens (c) kann auch so vorgegangen werden, dass man zunächst die Zwischenprodukte der Formel (V) herstellt und ohne deren Isolierung und ohne Lösungsmittelwechsel die weitere Umsetzung durchführt und die Endprodukte der Formel (I) im Rahmen eines Eintopfverfahrens in einem Arbeitsgang erhält.

Zur Herstellung von physiologisch verträglichen Säureadditionssalzen der Verbindungen der Formel (I) kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionellen Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäure, wie z.B. p-Toluolsulfonsäure, und 1,5-Naphthalindisulfonsäure.

Die Säureadditionssalze der Verbindungen der Formel (I) können in einfacher Weise nach üblichen Salzbildungsmethoden, z.B. durch Lösen einer Verbindung der Formel (I) in einem geeigneten organischen Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalzkomplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z.B. Chlorwasserstoffsäure und die Bromwasserstoffsäure, ferner Phosphorsäure, Salpetersäure und Schwefelsäure.

Die Metallkomplexe von Verbindungen der Formel (I) können in einfacher Weise nach üblichen Verfahren erhalten werden, so z.B. durch Lösen des Metallsalzes in Alkohol, wie z.B. Ethanol und Hinzufügen zur Verbindung der Formel (I). Man kann Metallsalzkomplexe in bekannter Weise, z.B. durch Abfiltrieren, Isolieren und gegebenenfalls durch Umkristallisieren reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromyzetes, Oomyzetes, Chytridiomyzetes, Zygomyzetes, Ascomyzetes, Basidiomyzetes, Deuteromyzetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemässen Wirkstoffe mit besonders gutem Erfolg zur Bekämpfung von Getreidekrankheiten, wie z.B. gegen den Erreger des Gerstenmehltaus *(Erysiphe graminis)* oder gegen die Erreger der Streifenkrankheit *(Drechslera graminea)* oder gegen die Erreger *Pyrenophora teres* und *Cochliobolus sativus*, zur Bekämpfung von Reiskrankheiten, wie z.B. *Pyricularia oryzae* oder *Pellicularia sasakii*, von Venturia-Arten, wie z.B. gegen den Erreger des Apfelschorfs *(Venturia inaequalis)* eingesetzt werden.

Die erfindungsgemäss verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung, bezogen auf das Entwicklungsstadium der Pflanze, sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern

von wirtschaftlichem Interesse, denn dadurch kann die Häufigkeit der Grasschnitte in Ziergärten, Park- und Sportanlagen, an Strassenrändern, auf Flughäfen oder in Obstanlagen reduziert werden. Von Bedeutung ist auch die Hemmung des Wuchses von krautigen und holzigen Pflanzen an Strassenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (Lagerns) der Pflanzen vor der Ernte verringert oder vollkommen beseitigt. Ausserdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne dass die Gefahr besteht, dass das Getreide lagert.

Eine Hemmung des vegetativen Wachstums ermöglicht bei vielen Kulturpflanzen eine dichtere Anpflanzung, so dass Mehrerträge bezogen auf die Bodenfläche erzielt werden können. Ein Vorteil der so erzielten kleineren Pflanzen ist auch, dass die Kultur leichter bearbeitet und beerntet werden kann.

Eine Hemmung des vegetativen Wachstums der Pflanzen kann auch dadurch zu Ertragssteigerungen führen, dass die Nährstoffe und Assimilate in stärkerem Masse der Blüten- und Fruchtbildung zugute kommen als den vegetativen Pflanzenteilen.

Mit Wachstumsregulatoren lässt sich häufig auch eine Förderung des vegetativen Wachstums erzielen. Dies ist von grossem Nutzen, wenn die vegetativen Pflanzenteile geerntet werden. Eine Förderung des vegetativen Wachstums kann aber auch gleichzeitig zu einer Förderung des generativen Wachstums führen, dadurch dass mehr Assimilate gebildet werden, so dass mehr oder grössere Früchte entstehen.

Ertragssteigerungen können in manchen Fällen durch einen Eingriff in den pflanzlichen Stoffwechsel erreicht werden, ohne dass sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zukker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Ausserdem lässt sich die Produktion oder der Abfluss von sekundären Pflanzeninhaltsstoffen positiv beeinflussen. Als Beispiel sei die Stimulierung des Latexflusses bei Gummibäumen genannt.

Unter dem Einfluss von Wachstumsregulatoren kann es zur Ausbildung parthenokarper Früchte kommen. Ferner kann das Geschlecht der Blüten beeinflusst werden. Auch kann eine Sterilität des Pollens erzeugt werden, was bei der Züchtung und Herstellung von Hybridsaatgut eine grosse Bedeutung hat.

Durch den Einsatz von Wachstumsregulatoren lässt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. grosses Interesse im Tabakanbau oder bei der Anpflanzung von Tomaten.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Baumwolle eine grosse Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden (Ausdünnung), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren lässt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen lässt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, dass z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflusst werden, so dass die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes

von Knospen oder der Keimung von Samen mit Hilfe von Wachstumsregulatoren kann in frostgefährdeten Gebieten erwünscht sein, um Schädigungen durch Spätfröste zu vermeiden.

Schliesslich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromate oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier- und/oder schaumerzeugende Mittel kommen in Frage: z.B. nichtionogene und anionische Emulgatoren, wie Polyoxyethylenfettsäureester, Polyoxyethylenfettalkoholether, z.B. Alkylarylpolyglycolether, Alkylsulfonate, Alkylsulfate, Arylsulfonate sowie Eiweisshydrolysate. Als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latex förmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90.

Die erfindungsgemässen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Giessen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemässen Verbindungen als Pflanzenwachstumsregulatoren können die Aufwandmengen in einem grösseren Bereich variiert werden. Im allgemeinen verwendet man pro Hektar Bodenfläche 0,01 bis 50, bevorzugt 0,05 bis 10 kg.

Beim Einsatz der erfindungsgemässen Stoffe als Pflanzenwachstumsregulatoren gilt, dass die Anwendung in einem bevorzugten Zeitraum vorgenommen wird, dessen genaue Abgrenzung sich nach klimatischen und vegetativen Gegebenheiten richtet.

Auch beim Einsatz der erfindungsgemässen Stoffe als Fungizide kann die Aufwandmenge je nach Art der Applikation in einem grösseren Bereich variiert werden. So liegen die Wirkungskonzentrationen bei der Behandlung von Pflanzenteilen in den Anwendungsformen im allgemeinen zwischen 1 und 0,0001, vorzugsweise zwischen 0,5 und 0,001 Gew.-%. Bei der Saatgutbehandlung werden im allgemeinen Wirkstoffmengen von 0,001 bis 50 g je Kilo Saatgut, vorzugsweise 0,01 bis 10, benötigt. Bei der Behandlung des Bodens sind Wirkstoffkonzentrationen von 0,00001 bis 0,1, vorzugsweise von 0,0001 bis 0,02 Gew.-%, am Wirkungsort erforderlich.

*Herstellungsbeispiele*

*Beispiel 1:*

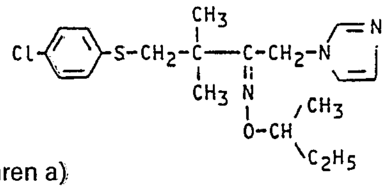

(Verfahren a)

30,9 (0,1 Mol) 4-(4-Chlorphenylsulfenyl)-3,3-dimethyl-1-(imidazol-1-yl)-butan-2-on werden zusammen mit 11,6 g (0,13 Mol) O-(2-Butyl)hydroxylamin und 2 g p-Toluolsulfonsäure in 600 ml Toluol 20 Stunden am Wasserabscheider unter Rückfluss erhitzt. Die Reaktionsmischung wird abgekühlt, mit Wasser gewaschen, über Natriumsulfat getrocknet und im Vakuum eingeengt. Der Rückstand wird säulenchromatographisch gereinigt.

Man erhält 10,6 g (28% der Theorie) 2-(2-Butyloximino)-4-(4-chlorphenylsulfenyl)-3,3-dimethyl-1-(imidazol-1-yl)butan als Öl vom Brechungsindex $n_D^{20}$ 1,5630.

*Herstellung des Ausgangsproduktes*

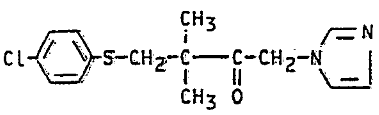

199 g (0,618 Mol) 1-Brom-4-(4-chlorphenylsulfenyl)-3,3-dimethylbutan-2-on, 120 g (1,76 Mol) Imidazol und 243,5 g (1,76 Mol) Kaliumcarbonat in 3 l Aceton werden 5 Stunden unter Rückfluss gerührt. Danach lässt man abkühlen, saugt die anorganischen Salze ab und engt das Filtrat ein. Der Rückstand wird in Methylenchlorid aufgenommen, dreimal mit Wasser gewaschen, über Natriumsulfat getrocknet und eingeengt. Nach Umkristallisation aus Diisopropylether erhält man 156 g (82% der Theorie) 4-(4-Chlorphenylsulfenyl)-3,3-dimethyl-1-(imidazol-1-yl)butan-2-on vom Schmelzpunkt 50°C.

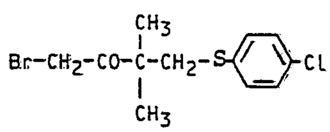

97 g (0,4 Mol) 1-(4-Chlorphenylsulfenyl)-2,2-dimethylbutan-3-on werden bei Raumtemperatur langsam mit 64 g (0,4 Mol) Brom versetzt. Das Reaktionsgemisch wird entsprechend Beispiel 1 aufgearbeitet. Man erhält 127 g (99% der Theorie) 1-Brom-4-(4-chlorphenylsulfenyl)-3,3-dimethylbutan-2-on als zähflüssiges Öl.

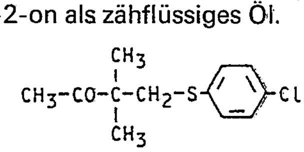

134,5 g (1 Mol) 4-Chlorpinakolon werden mit 216 g (1,5 Mol) 4-Chlorthiophenol und 210 g (1,52 Mol) Kaliumcarbonat in 500 ml Dimethylformamid 15 Stunden bei 150°C und einem Druck von 2 bis 4 bar gerührt. Man lässt auf Raumtemperatur abkühlen, verrührt mit 10 l Wasser und extrahiert mit Ether. Die Etherphase wird über Natriumsulfat getrocknet, eingeengt und der Rückstand im Vakuum destilliert. Man erhält 151 g (62% der Theorie) 1-(4-Chlorphenylsulfenyl)-2,2-dimethylbutan-3-on vom Siedepunkt 146°C/0,7 mbar.

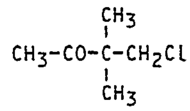

11,6 g (0,1 Mol) 2,2-Dimethyl-1-hydroxybutan-3-on (Herstellung, vgl. Beispiel 3) werden bei 50 bis 60°C (Eiskühlung) zu 20,5 g (0,1 Mol) N,N-dimethyl-1,2,2-trichlorvinylamin getropft. Nach zweistündigem Rühren bei 60°C wird im Wasserstrahlvakuum destilliert. Man erhält 8,1 g (60% der Theorie) 4-Chlorpinakolin vom Siedepunkt 60-62°C/16 mbar.

*Beispiel 2:*

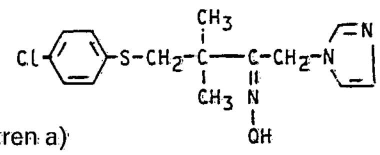

(Verfahren a)

30,9 g (0,1 Mol) 4-(4-Chlorphenylsulfenyl)-3,3-dimethyl-1-(imidazol-1-yl)butan-2-on werden zusammen mit 20 g (0,13 Mol) Hydroxylaminhydrochlorid, 11,8 g (0,13 Mol) Triethylamin und 2 g p-Toluolsulfonsäure in 200 ml Toluol 4 Stunden unter Rückfluss am Wasserabscheider erhitzt. Die Reaktionsmischung wird abgekühlt, mit Wasser gewaschen, über Natriumsulfat getrocknet und im Vakuum eingeengt. Der Rückstand wird säulenchromatographisch gereinigt.

Man erhält 13,6 g (42% der Theorie) 4-(4-Chlorphenylsulfenyl)-3,3-dimethyl-1-(imidazol-1-yl)-2-oximinobutan vom Schmelzpunkt 125-126°C.

*Beispiel 3:*

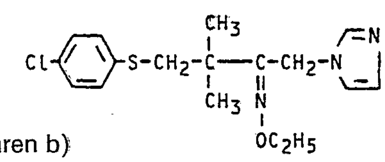

(Verfahren b)

13,0 g (0,04 Mol) 4-(4-Chlorphenylsulfenyl)-3,3-dimethyl-1-(imidazol-1-yl)-2-oximinobutan werden in 50 ml Dimethylformamid gelöst und mit 4,5 g (0,04 Mol) Natrium-t-butylat versetzt. Nach Erwärmen der Mischung auf 50°C tropft man 6,9 g (0,044 Mol) Jodethan zu und rührt 2 Stunden bei 50°C. Die Reaktionsmischung wird abgekühlt, mit 600 ml Wasser verrührt und mit Essigester extrahiert. Die Essigesterphase wird dreimal mit Wasser gewaschen, über Natriumsulfat getrocknet und im Vakuum bei 50°C Badtemperatur eingeengt. Der Rückstand wird säulenchromatographisch gereinigt. Man erhält 12,1 g (86,4% der Theorie) 4-(4-Chlorphenylsulfenyl)-3,3-dimethyl-2-ethyloximino-1-(imidazol-1-yl)butan als Öl vom Brechungsindex $n_D^{20}$ 1,5699.

In entsprechender Weise und gemäss den angegebenen Verfahren werden die folgenden Verbindungen der allgemeinen Formel (I):

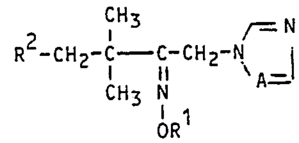 (I)

erhalten:

| Beispiel Nr. | $R^1$ | $R^2$ | A | Schmelzpunkt (°C) bzw. Brechungsindex ($n_D^{20}$) |
|---|---|---|---|---|
| 4 | $CH_3$ | 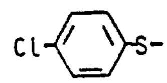 | CH | 1,5835 |
| 5 | $CH_3$ | 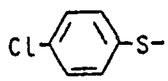 | CH | 118-120 (×HCl) |
| 6 | $CH_3$ | 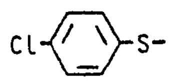 | CH | 158 (×$CuCl_2$) |
| 7 | n-$C_3H_7$ | 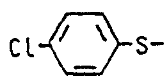 | CH | 1,5665 |
| 8 | i-$C_3H_7$ | 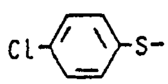 | CH | 1,5632 |
| 9 | n-$C_4H_9$ | 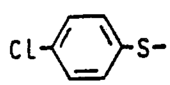 | CH | 1,5567 |
| 10 | n-$C_7H_{15}$ | 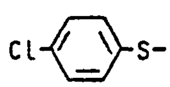 | CH | 1,5419 |
| 11 | H | 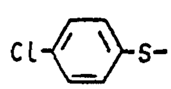 | CH | 1,5730 |
| 12 | H | 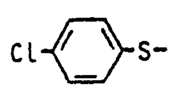 | CH | 1,5522 |
| 13 | $CH_2{=}CH{-}CH_2{-}$ | 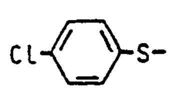 | CH | 1,5790 |
| 14 | $HC{\equiv}C{-}CH_2{-}$ | 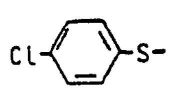 | CH | 1,5849 |

| Beispiel Nr. | $R^1$ | $R^2$ | A | Schmelzpunkt (° C) bzw. Brechungsindex ($n_D^{20}$) |
|---|---|---|---|---|
| 15 | $CH_3-CH=CH-CH_2-$ | 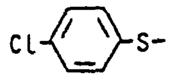 | CH | 1,5722 |
| 16 | 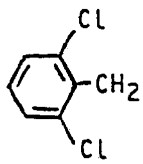 | 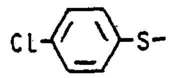 | CH | 1,5997 |
| 17 | H | 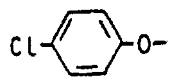 | CH | 175-176 (×HCl) |
| 18 | $C_2H_5$ | 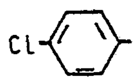 | CH | 1,5454 |
| 19 | n-$C_3H_7$ | 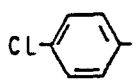 | CH | 1,5431 |
| 20 | n-$C_4H_9$ | 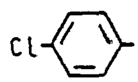 | CH | 1,5400 |
| 21 | $CH_3$ | 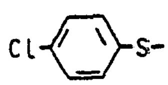 | N | 1,5776 |
| 22 | sek.-$C_4H_9$ | 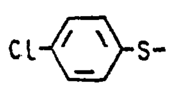 | N | 1,5523 (Form A) * |
| 23 | sek.-$C_4H_9$ | 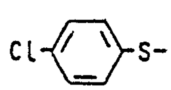 | N | 1,5496 (Form B) * |
| 24 | n-$C_7H_{15}$ | 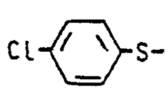 | N | 1,5334 |

* Formen A und B: die beiden möglichen geometrischen Isomeren

| Beispiel Nr. | $R^1$ | $R^2$ | A | Schmelzpunkt (0° C) bzw. Brechungsindex ($n_D^{20}$) |
|---|---|---|---|---|
| 25 | 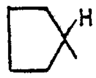 | 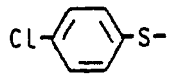 | N | 1,5673 |
| 26 | 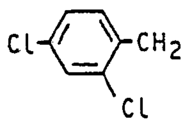 | 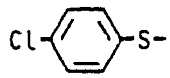 | N | 1,5961 |
| 27 | 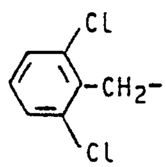 | 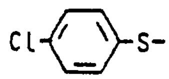 | N | 1,5850 |
| 28 | n-$C_4H_9$ | 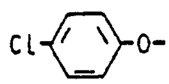 | N | 1,5170 |
| 29 | 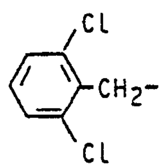 | 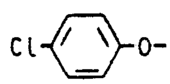 | N | 165-170 (×HCl) |
| 30 | 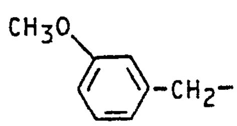 | 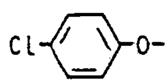 | N | 114 (×HCl) |
| 31 | n-$C_3H_7$ | 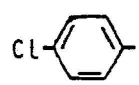 | N | 42 |
| 32 | i-$C_3H_7$ | 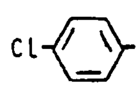 | N | 66 |
| 33 | n-$C_7H_{15}$ | 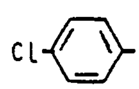 | N | 1,5185 |
| 34 | 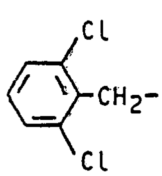 | 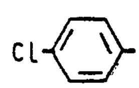 | N | 1,5779 |

| Beispiel Nr. | $R^1$ | $R^2$ | A | Schmelzpunkt (° C) bzw. Brechungsindex ($n_D^{20}$) |
|---|---|---|---|---|
| 35 | 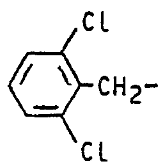 | 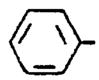 | N | 1,5610 |
| 36 | sek.-$C_4H_9$ | 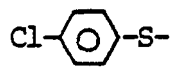 | N | 1,5496 |
| 37 | H | 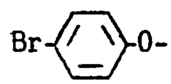 | N | Fp. 168-190° C (×HCl) |
| 38 | H | 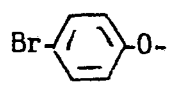 | CH | Fp. 98-100° C |
| 39 | H | 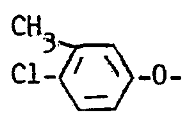 | N | Fp. 93-95° C |
| 40 | n-$C_4H_9$ | 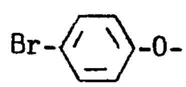 | N | $n_D^{25}$ 1,5363 |
| 41 | $CH_3$ | 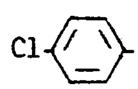 | CH | zähes Öl |
| 42 | n-$C_3H_7$– | 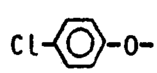 | N | 1,5234 |
| 43 | $CH_3-CH=CH-CH_2-$ | 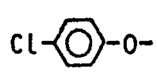 | N | 1,5205 |
| 44 | $CH_3-CH=CH-CH_2-$ | 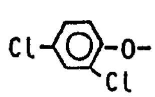 | N | 1,5379 |

| Beispiel Nr. | $R^1$ | $R^2$ | A | Schmelzpunkt (°C) bzw. Brechungsindex ($n_D^{20}$) |
|---|---|---|---|---|
| 45 | $CH_2=CH-CH_2-$ | 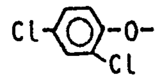 | N | 1,5325 |
| 46 | $C_2H_5-$ | 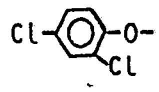 | N | 1,5353 |
| 47 | $C_2H_5-$ | 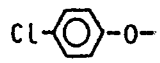 | N | 1,5329 |
| 48 | $n\text{-}C_3H_7-$ | 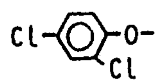 | N | 1,5264 |
| 49 | $n\text{-}C_4H_9-$ | 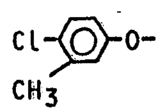 | N | 1,5262 |
| 50 | H | 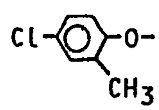 | N | 208 (×HCl) |
| 51 | $CH_2=CH-CH_2-$ | 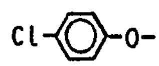 | N | $n_D^{22}$ 1,5352 |
| 52 | $n\text{-}C_4H_9-$ | 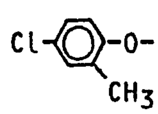 | N | $n_D^{20,8}$ 1,5208 |
| 53 | $C_2H_5$ | 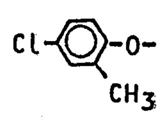 | N | $n_D^{20,7}$ 1,5278 |
| 54 | H | 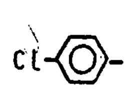 | CH | 153 |

| Beispiel Nr. | $R^1$ | $R^2$ | A | Schmelzpunkt (0° C) bzw. Brechungsindex ($n_D^{20}$) |
|---|---|---|---|---|
| 55 | $HC\equiv C-CH_2-$ | 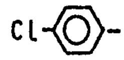 | CH | 1,5574 |
| 56 | $CH_3-CH=CH-CH_2-$ | 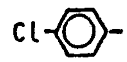 | CH | 1,5493 |
| 57 | H | 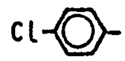 | N | 135-138 |
| 58 | $HC\equiv C-CH_2-$ | 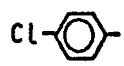 | N | 1,5511 |
| 59 | $CH_2=CH-CH_2-$ | 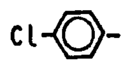 | N | 1,5431 |
| 60 | H | 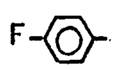 | CH | 145 |
| 61 | $CH_3$ | 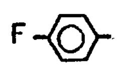 | CH | 1,5365 |
| 62 | $n-C_4H_9$ | 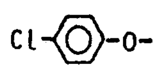 | CH | $n_D^{21,8}$ 1,5270 |

*Anwendungsbeispiele*

In den nachfolgenden Anwendungsbeispielen werden die nachstehend angegebenen Verbindungen als Vergleichssubstanzen eingesetzt:

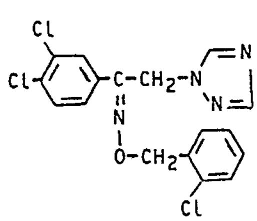
(A)

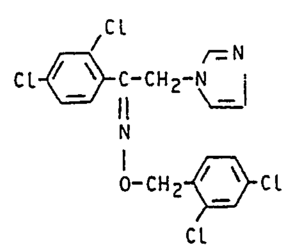
x $HNO_3$ (B)

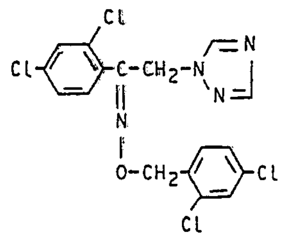

x $HNO_3$ (C)

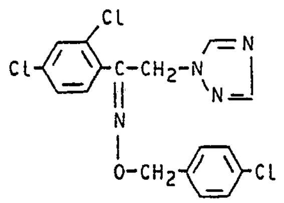

x $HNO_3$ (D)

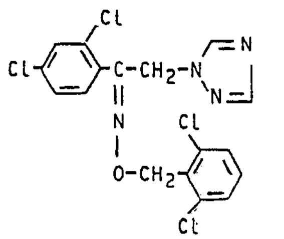

(E)

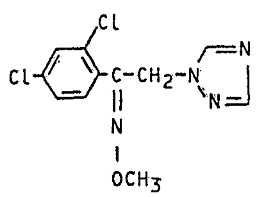

x $HNO_3$ (F)

*Beispiel A:*

Erysiphe-*Test (Gerste)/protektiv*

Lösungsmittel: 100 Gewichtsteile Dimethylformamid

Emulgator: 0,25 Gewichtsteile Alkylarylpolyglykolether

Zur Herstellung einer zweckmässigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von *Erysiphe graminis* f.sp. *hordei* bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäss folgenden Herstellungsbeispielen: 8, 9, 11, 12, 16, 17, 22, 23, 24, 25, 26, 27, 28, 30.

*Beispiel B:*

Pyricularia-*Test (Reis)/protektiv*

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wässerigen Sporensuspension von *Pyricularia oryzae* inokuliert. Anschliessend werden die Pflanzen in einem Gewächshaus bei 100% relativer Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z.B. die Verbindungen gemäss folgenden Herstellungsbeispielen: 7, 23.

*Beispiel C:*

Pellicularia-*Test (Reis)*

Lösungsmittel: 12,5 Gewichtsteile Aceton

Emulgator: 0,3 Gewichtsteile Alkylarylpolyglykolether

Zur Herstellung einer zweckmässigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnass gespritzt. Die Pflanzen verbleiben bis zum Abtrocknen im Gewächshaus. Anschliessend werden die Pflanzen mit *Pellicularia sasakii* inokuliert und bei 25°C und 100% relativer Luftfeuchtigkeit aufgestellt.

5-8 Tage nach der Inokulation erfolgt die Auswertung des Krankheitsbefalls.

Eine deutliche Überlegenheit in der Wirksamkeit gegenüber dem Stand der Technik zeigt bei diesem Test z.B. die Verbindung gemäss folgendem Herstellungsbeispiel: 7.

*Beispiel D:*

Drechslera graminea-*Test (Gerste)/Saatgutbehandlung (syn.* Helminthosporium gramineum*)*

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmässige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossene Petrischalen im Kühlschrank 10 Tage lang einer Temperatur von 4°C aus. Dabei wird die Keimung der Gerste und gegebenenfalls auch der Pilzsporen eingeleitet. Anschliessend sät man die vorgekeimte Gerste mit 2×50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigt bei diesem Test z.B. die Verbindung gemäss folgendem Herstellungsbeispiel: 17.

*Beispiel E:*

*Wuchshemmung bei Baumwolle*

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 22, 25, 31, 32, 33 zeigen in diesem Test eine deutliche Überlegenheit in der Wirksamkeit gegenüber dem Stand der Technik.

*Beispiel F:*

*Wuchshemmung bei Sojabohnen*

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnass mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

Die erfindungsgemässen Wirkstoffe 22, 25, 28, 31, 32 zeigen in diesem Test eine gute Wuchsbeeinflussung im Vergleich zur Kontrolle.

*Beispiel G:*

*Wuchsbeeinflussung bei Zuckerrüben*

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnass mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeutet 0% Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die erfindungsgemässen Wirkstoffe 22, 23, 24, 25, 31 und 32 zeigen in diesem Test eine starke Wuchsbeeinflussung.

*Beispiel H:*

*Stimulierung der $CO_2$-Fixierung bei Sojabohnen*

Lösungsmittel: 30 Gewichtsteile Dimethylformamid

Emulgator: 1 Gewichtsteil Polyoxyethylen-Sorbitan-Monolaurat

Zur Herstellung einer zweckmässigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnass mit den Wirkstoffzubereitungen besprüht. Im weiteren Verlauf des Experimentes wird die $CO_2$-Fixierung der Pflanzen mit üblichen Methoden gemessen. Die Werte werden mit denen nicht mit Wirkstoffen behandelter Kontrollpflanzen verglichen.

## Patentansprüche

1. 1-Azolyl-2-oximinobutanderivate der allgemeinen Formel:

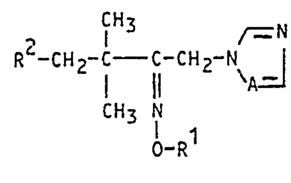

(I)

in welcher

A für Stickstoff oder die CH-Gruppe steht,

$R^1$ für Wasserstoff, für geradkettiges oder verzweigtes Alkyl mit 1 bis 12 Kohlenstoffatomen, für geradkettiges oder verzweigtes Alkenyl oder Alkinyl mit jeweils 2 bis 12 Kohlenstoffatomen, für geradkettiges oder verzweigtes Halogenalkyl mit 1 bis 4 Kohlenstoffatomen und 1 bis 5 gleichen oder verschiedenen Halogenatomen steht, ferner für Cycloalkyl oder Cycloalkenyl mit jeweils 5 bis 7 Kohlenstoffatomen, Cycloalkylalkyl mit 3 bis 7 Kohlenstoffatomen im Cycloalkylteil und 1 bis 4 Kohlenstoffatomen im Alkylteil, und schliesslich für gegebenenfalls einfach oder mehrfach, gleich oder verschieden substituiertes Benzyl oder Phenyl steht, wobei als Substituenten Halogen, Cyano, Nitro, Alkyl und Alkoxy mit jeweils 1 bis 4 Kohlenstoffatomen, gegebenenfalls durch Halogen substituiertes Phenyl oder Phenoxy, Halogenalkyl oder Halogenalkoxy mit jeweils 1 bis 3 gleichen oder verschiedenen Halogenatomen zu nennen sind, und

$R^2$ für jeweils gegebenenfalls einfach oder mehrfach, gleich oder verschieden substituiertes Phenyl, Phenoxy, Phenylthio, Phenylsulfinyl oder Phenylsulfonyl steht, wobei als Substituenten Halogen, Nitro, Cyano, ferner Alkyl, Alkoxy oder Alkylthio mit jeweils 1 bis 6 Kohlenstoffatomen, weiterhin Cyclohexyl, sodann Halogenalkyl, Halogenalkoxy und Halogenalkylthio mit jeweils 1 bis 4 Kohlenstoffatomen und 1 bis 5 gleichen oder verschiedenen Halogenatomen, ausserdem Dialkylamino mit jeweils 1 bis 4 Kohlenstoffatomen in jedem Alkylteil, sodann Alkoxycarbonyl mit 1 bis 4 Kohlenstoffatomen im Alkylteil, sowie schliesslich jeweils gegebenenfalls einfach oder mehrfach durch Halogen und/oder Alkyl mit 1 bis 4 Kohlenstoffatomen substituiertes Phenyl, Phenoxy, Phenylthio, Benzyl, Benzoxy oder Benzylthio zu nennen sind,

sowie deren physiologisch verträgliche Säureadditionssalze und Metallkomplexe.

2. Verbindungen der allgemeinen Formel (I) in Anspruch 1, wobei

$R^1$ für geradkettiges oder verzweigtes Alkyl mit 1 bis 8 Kohlenstoffatomen, für geradkettiges oder verzweigtes Alkenyl oder Alkinyl mit jeweils 2 bis 8 Kohlenstoffatomen steht, ferner für Cyclopentyl, Cyclohexyl, Cyclopropylmethyl, Cyclopropylethyl, Cyclopentylmethyl, Cyclohexylmethyl sowie für gegebenenfalls 1- bis 3fach, gleich oder verschieden substituiertes Benzyl oder Phenyl steht, wobei als Substituenten genannt seien: Fluor, Chlor, Brom, Cyano, Nitro, Methyl, Ethyl, Methoxy, gegebenenfalls durch Fluor oder Chlor substituiertes Phenyl oder Phenoxy sowie Trifluormethyl und Trifluormethoxy, und

$R^2$ für jeweils gegebenenfalls 1- oder 2fach, gleich oder verschieden substituiertes Phenyl, Phenoxy, Phenylthio, Phenylsulfinyl oder Phenylsulfonyl steht, wobei als Substituenten genannt seien: Fluor, Chlor, Brom, Methyl, gegebenenfalls durch Fluor, Chlor oder Methyl substituiertes Phenyl oder Phenoxy, und

A die in Anspruch 1 angegebene Bedeutung besitzt.

3. Verfahren zur Herstellung von 1-Azolyl-2-oximinobutanderivate der allgemeinen Formel:

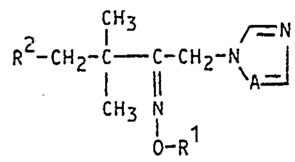

(I)

in welcher

A für Stickstoff oder die CH-Gruppe steht,

$R^1$ für Wasserstoff, für geradkettiges oder verzweigtes Alkyl mit 1 bis 12 Kohlenstoffatomen, für geradkettiges oder verzweigtes Alkenyl oder Alkinyl mit jeweils 2 bis 12 Kohlenstoffatomen, für geradkettiges oder verzweigtes Halogenalkyl mit 1 bis 4 Kohlenstoffatomen und 1 bis 5 gleichen oder verschiedenen Halogenatomen steht, ferner für Cycloalkyl oder Cycloalkenyl mit jeweils 5 bis 7 Kohlenstoffatomen, Cycloalkylalkyl mit 3 bis 7 Kohlenstoffatomen im Cycloalkylteil und 1 bis 4 Kohlenstoffatomen im Alkylteil, und schliesslich für gegebenenfalls einfach oder mehrfach, gleich oder verschieden substituiertes Benzyl oder Phenyl steht, wobei als Substituenten Halogen, Cyano, Nitro, Alkyl und Alkoxy mit jeweils 1 bis 4 Kohlenstoffatomen, gegebenenfalls durch Halogen substituiertes Phenyl oder Phenoxy, Halogenalkyl oder Halogenalkoxy mit jeweils 1 bis 3 gleichen oder verschiedenen Halogenatomen zu nennen sind, und

$R^2$ für jeweils gegebenenfalls einfach oder mehrfach, gleich oder verschieden substituiertes Phenyl, Phenoxy, Phenylthio, Phenylsulfinyl oder Phenylsulfonyl steht, wobei als Substituenten Halogen, Nitro, Cyano, ferner Alkyl, Alkoxy oder Alkylthio mit jeweils 1 bis 6 Kohlenstoffatomen, weiterhin Cyclohexyl, sodann Halogenalkyl, Halogenalkoxy und Halogenalkylthio mit jeweils 1 bis 4 Kohlenstoffatomen und 1 bis 5 gleichen oder verschiedenen Halogenatomen, ausserdem Dialkylamino mit jeweils 1 bis 4 Kohlenstoffatomen in jedem Alkylteil, sodann Alkoxycarbonyl mit 1 bis 4 Kohlenstoffatomen im Alkylteil, schliesslich jeweils gegebenenfalls einfach oder mehrfach durch Halogen und/oder Alkyl mit 1 bis 4 Kohlenstoffatomen substituiertes Phenyl, Phenoxy, Phenylthio, Benzyl, Benzoxy oder Benzylthio zu nennen sind,

dadurch gekennzeichnet, dass man

a) Azolylketone der Formel (II):

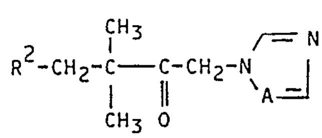

(II)

in welcher A und $R^2$ die oben angegebene Bedeutung haben, mit substituierten Hydroxylaminen der Formel (III):

$$H_2N-O-R^1 \quad (III)$$

in welcher $R^1$ die oben angegebene Bedeutung

hat, gegebenenfalls in Gegenwart eines Verdünnungsmittels umsetzt; oder

b) die erfindungsgemässen 1-Azolyl-2-oximinobutanderivate der allgemeinen Formel (Ia):

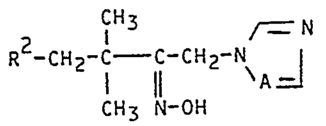

(Ia)

in welcher A und $R^2$ die oben angegebene Bedeutung haben,

mit Halogeniden der Formel (IV):

$$Hal-R^1 \quad (IV)$$

in welcher

$R^1$ die oben angegebene Bedeutung hat, und

Hal für Chlor oder Brom steht,

gegebenenfalls in Gegenwart einer starken Base und in Gegenwart eines Verdünnungsmittels umsetzt, oder

c) 1-Halogen-2-oximinobutanderivate der allgemeinen Formel (V):

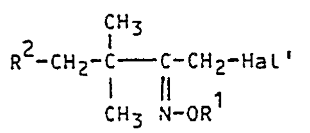

(V)

in welcher

$R^1$ und $R^2$ die oben angegebene Bedeutung haben, und Hal' für Chlor oder Brom steht, mit 1,2,4-Triazol oder Imidazol in Gegenwart eines Säurebindemittels und in Gegenwart eines Verdünnungsmittels umsetzt, und noch gegebenenfalls an die so erhaltenen Verbindungen der Formel (I) anschliessend eine Säure oder ein Metallsalz addiert.

4. Pflanzenschutzmittel, gekennzeichnet durch einen Gehalt an mindestens einem 1-Azolyl-2-oximinobutanderivat der Formel (I) in Ansprüchen 1 und 3.

5. Fungizide und das Wachstum von Pflanzen regulierende Mittel, gekennzeichnet durch einen Gehalt an mindestens einem 1-Azolyl-2-oximinobutanderivat der Formel (I) in Ansprüchen 1 und 3.

6. Verfahren zur Bekämpfung von Pilzen, dadurch gekennzeichnet, dass man 1-Azolyl-2-oximinobutanderivat der Formel (I) in Ansprüchen 1 und 3 auf Pilze oder ihren Lebensraum einwirken lässt.

7. Verfahren zur Regulierung des Pflanzenwachstums, dadurch gekennzeichnet, dass man 1-Azolyl-2-oximinobutanderivate der Formel (I) in Ansprüchen 1 und 3 auf Pflanzen und ihren Lebensraum einwirken lässt.

8. Verwendung von 1-Azolyl-2-oximinobutanderivaten der Formel (I) in Ansprüchen 1 und 3 zur Bekämpfung von Pilzen und zur Regulierung des Pflanzenwachstums.

## Claims

1. 1-Alzolyl-2-oximinobutane derivatives of the general formula

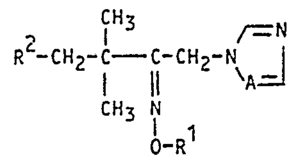

(I)

in which

A represents nitrogen or the CH group,

$R^1$ represents hydrogen, straight-chain or branched alkyl with 1 to 12 carbon atoms, straight-chain or branched alkenyl or alkinyl each with 2 to 12 carbon atoms, straight-chain or branched halogenoalkyl with 1 to 4 carbon atoms and 1 to 5 identical or different halogen atoms, also represents cycloalkyl or cycloalkenyl each with 5 to 7 carbon atoms, cycloalkylalkyl with 3 to 7 carbon atoms in the cycloalkyl part and 1 to 4 carbon atoms in the alkyl part, and finally represents phenyl or benzyl optionally mono- or polysubstituted by identical or different substituents, the following being mentioned as substituents: halogen, cyano, nitro, alkyl and alkoxy each with 1 to 4 carbon atoms, optionally halogen-substituted phenyl or phenoxy, halogenoalkyl or halogenoalkoxy each with 1 to 3 identical or different halogen atoms, and

$R^2$ represents phenyl, phenoxy, phenylthio, phenylsulphinyl or phenylsulphonyl, each optionally mono- or polysubstituted by identical or different substituents, the following being mentioned as substituents: halogen, nitro or cyano, also alkyl, alkoxy or alkylthio each with 1 to 6 carbon atoms, furthermore cyclohexyl, then halogenoalkyl, halogenoalkoxy and halogenoalkylthio each with 1 to 4 carbon atoms and 1 to 5 identical or different halogen atoms, in addition dialkylamino with in each case 1 to 4 carbon atoms in each alkyl part, then alkoxycarbonyl with 1 to 4 carbon atoms in the alkyl part, and finally phenyl, phenoxy, phenylthio, benzyl, benzoxy or benzylthio, each optionally mono- or polysubstituted by halogen and/or alkyl with 1 to 4 carbon atoms, and physiologically tolerated acid addition salts and metal complexes thereof.

2. Compounds of the general Formula (I) in Claim 1, wherein

$R^1$ represents straight-chain or branched alkyl with 1 to 8 carbon atoms, straight-chain or branched alkenyl or alkinyl each with 2 to 8 carbon atoms, also represents cyclopentyl, cyclohexyl, cyclopropylmethyl, cyclopropylethyl, cyclopentylmethyl, cyclohexylmethyl and phenyl or benzyl optionally mono- to trisubstituted by identical or different substituents, the following being mentioned as substituents: fluorine, chlorine, bromine, cyano, nitro, methyl, ethyl, methoxy, optionally fluorine- or chlorine-substituted phenyl or phenoxy and trifluoromethyl and trifluoromethoxy, and

$R^2$ represents phenyl, phenoxy, phenylthio, phenylsulphinyl or phenylsulphonyl, each optionally mono- or disubstituted by identical or different substituents, the following being mentioned as substituents: fluorine, chlorine, bromine, methyl and optionally fluorine-, chlorine- or methylsubstituted phenyl or phenoxy, and

A has the meaning given in Claim 1.

3. Process for the preparation of 1-azolyl-2-oximinobutane derivatives of the general formula:

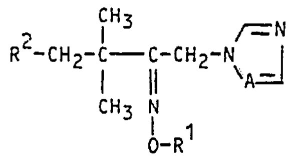

(I)

in which

A represents nitrogen or the CH group, $R^1$ represents hydrogen, straight-chain or branched alkyl with 1 to 12 carbon atoms, straight-chain or branched alkenyl or alkinyl each with 2 to 12 carbon atoms, straight-chain or branched halogenoalkyl with 1 to 4 carbon atoms and 1 to 5 identical or different halogen atoms, also represents cycloalkyl or cycloalkenyl each with 5 to 7 carbon atoms, cycloalkylalkyl with 3 to 7 carbon atoms in the cycloalkyl part and 1 to 4 carbon atoms in the alkyl part, and finally represents phenyl or benzyl optionally mono- or polysubstituted by identical or different substituents, the following being mentioned as substituents: halogen, cyano, nitro, alkyl and alkoxy each with 1 to 4 carbon atoms, optionally halogensubstituted phenyl or phenoxy, halogenoalkyl or halogenoalkoxy each with 1 to 3 identical or different halogen atoms, and

$R^2$ represents phenyl, phenoxy, phenylthio, phenylsulphinyl or phenylsulphonyl, each optionally mono- or polysubstituted by identical or different substituents, the following being mentioned as substituents: halogen, nitro or cyano, also alkyl, alkoxy or alkylthio each with 1 to 6 carbon atoms, furthermore cyclohexyl, then halogenoalkyl, halogenoalkoxy and halogenoalkylthio each with 1 to 4 carbon atoms and 1 to 5 identical or different halogen atoms, in addition dialkylamino with in each case 1 to 4 carbon atoms in each alkyl part, then alkoxycarbonyl with 1 to 4 carbon atoms in the alkyl part, and finally phenyl, phenoxy, phenylthio, benzyl, benzoxy or benzylthio, each optionally mono- or polysubstituted by halogen and/or alkyl with 1 to 4 carbon atoms, characterised in that

(*a*) azolyl ketones of the Formula (II):

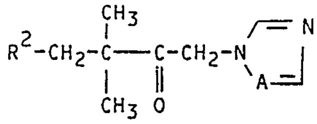

(II)

in which

A and $R^2$ have the above-mentioned meaning, are reacted with substituted hydroxylamines of the Formula (III):

$$H_2N-O-R^1 \qquad (III)$$

in which

$R^1$ has the above-mentioned meaning,

if appropriate in the presence of a diluent, or

(*b*) the 1-azolyl-2-oximinobutane derivatives according to the invention, of the general Formula (Ia):

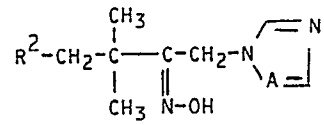

(Ia)

in which

A and $R^2$ have the above-mentioned meaning, are reacted with halides of the Formula (IV):

$$Hal-R^1 \qquad (IV)$$

in which

$R^1$ has the above-mentioned meaning, and

Hal represents chlorine or bromine,

if appropriate in the presence of a strong base and in the presence of a diluent, or

(*c*) 1-halogeno-2-oximinobutane derivatives of the general Formula (V):

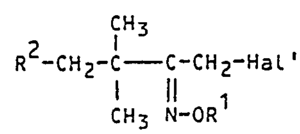

(V)

in which

$R^1$ and $R^2$ have the above-mentioned meaning, and Hal′ represents chlorine or bromine,

are reacted with 1,2,4-triazole or imidazole in the presence of an acid-binding agent and in the presence of a diluent and, if desired, an acid or a metal salt is then also added on to the compounds of the Formula (I) thus obtained.

4. Plant-protecting agents, characterised in that they contain at least one 1-azolyl-2-oximinobutane derivative of the Formula (I) in Claims 1 and 3.

5. Fungicides and agents regulating the growth of plants, characterised in that they contain at least one 1-azolyl-2-oximinobutane derivative of the Formula I in Claims 1 and 3.

6. Process for combating fungi, characterised in that 1-azolyl-2-oximinobutane derivatives of the Formula (I) in Claims 1 and 3 are allowed to act on fungi or their habitat.

7. Process for regulating plant growth, characterised in that 1-azolyl-2-oximinobutane derivatives of the Formula (I) in Claims 1 and 3 are allowed to act on plants and their habitat.

8. Use of 1-azolyl-2-oximinobutane derivatives of the Formula (I) in Claims 1 and 3 for combating fungi and for regulating plant growth.

**Revendications**

1. 1-Azolyl-2-oximinobutanes de formule générale:

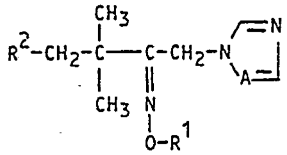

(I)

dans laquelle:

A représente l'azote ou le groupe CH,

$R^1$ représente l'hydrogène, un groupe alkyle à chaîne droite ou à chaîne ramifiée ayant 1 à 12 atomes de carbone, un groupe alcényle ou alcynyle à chaîne droite ou à chaîne ramifiée ayant chacun 2 à 12 atomes de carbone, un groupe halogénalkyle à chaîne droite ou à chaîne ramifiée ayant 1 à 4 atomes de carbone et 1 à 5 atomes d'halogènes égaux ou différents, en outre, un groupe cycloalkyle ou un groupe cycloalcényle ayant chacun 5 à 7 atomes de carbone, un groupe cycloalkylalkyle ayant 3 à 7 atomes de carbone dans la partie cycloalkyle et 1 à 4 atomes de carbone dans la partie alkyle, et finalement un groupe benzyle ou phényle portant, le cas échéant, un ou plusieurs substituants identiques ou différents, et on peut alors mentionner comme substituants un halogène, un groupe cyano, nitro, alkyle et alcoxy ayant chacun 1 à 4 atomes de carbone, phényle ou phénoxy éventuellement substitué par un halogène, halogénalkyle ou halogénalcoxy ayant chacun 1 à 3 atomes d'halogènes identiques ou différents, et

$R^2$ représente un groupe phényle, phénoxy, phénylthio, phénylsulfinyle ou phénylsulfonyle, chacun portant éventuellement un ou plusieurs substituants identiques ou différents, et on peut alors mentionner comme substituants un halogène, un groupe nitro, cyano, en outre, un groupe alkyle, alcoxy ou alkylthio ayant dans chaque cas 1 à 6 atomes de carbone, ainsi qu'un groupe cyclohexyle, en outre, un groupe halogénalkyle, un groupe halogénalcoxy et un groupe halogénalkylthio ayant chacun 1 à 4 atomes de carbone et 1 à 5 atomes d'halogènes identiques ou différents, en outre, un groupe dialkylamino ayant 1 à 4 atomes de carbone dans la partie alkyle, en outre, un groupe alcoxycarbonyle ayant 1 à 4 atomes de carbone dans la partie alkyle, ainsi que finalement un groupe phényle, phénoxy, phénylthio, benzyle, benzoxy ou benzylthio portant chacun, le cas échéant, un ou plusieurs substituants halogéno et/ou alkyle ayant 1 à 4 atomes de carbone,
de même que leurs sels d'addition d'acides physiologiquement acceptables et leurs complexes métalliques.

2. Composés de formule générale (I) suivant la revendication 1, dans laquelle:

$R^1$ désigne un groupe alkyle à chaîne droite ou à chaîne ramifiée ayant 1 à 8 atomes de carbone, un groupe alcényle ou alcynyle à chaîne droite ou à chaîne ramifiée ayant chacun 2 à 8 atomes de carbone, en outre le groupe cyclopentyle, cyclohexyle, cyclopropylméthyle, cyclopropyléthyle, cyclopentylméthyle, cyclohexylméthyle, de même qu'un groupe benzyle ou phényle portant éventuellement 1 à 3 substituants identiques ou différents, et on mentionne alors comme substituants: le fluor, le chlore, le brome, un groupe cyano, nitro, méthyle, éthyle, méthoxy, un groupe phényle ou phénoxy éventuellement substitués par du fluor ou du chlore, ainsi qu'un groupe trifluorméthyle et trifluorméthoxy, et

$R^2$ désigne un groupe phényle, phénoxy, phénylthio, phénylsulfinyle ou phénylsulfonyle, portant chacun, le cas échéant, 1 ou 2 substituants identiques ou différents, et on mentionne alors comme substituants: le fluor, le chlore, le brome, le groupe méthyle ainsi qu'un groupe phényle ou phénoxy, éventuellement substitués par du fluor, du chlore ou un groupe méthyle, et

A a la définition indiquée dans la revendication 1.

3. Procédé de production de 1-azolyl-2-oximinobutanes de formule générale:

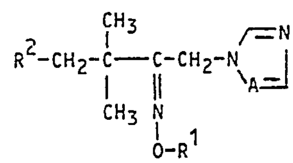

(I)

dans laquelle:

A représente l'azote ou le groupe CH,

$R^1$ représente l'hydrogène, un groupe alkyle à chaîne droite ou à chaîne ramifiée ayant 1 à 12 atomes de carbone, un groupe alcényle ou alcynyle à chaîne droite ou à chaîne ramifiée ayant chacun 2 à 12 atomes de carbone, un groupe halogénalkyle à chaîne droite ou à chaîne ramifiée, ayant 1 à 4 atomes de carbone et 1 à 5 atomes d'halogènes égaux ou différents, en outre, un groupe cycloalkyle ou un groupe cycloalcényle ayant chacun 5 à 7 atomes de carbone, un groupe cycloalkylalkyle ayant 3 à 7 atomes de carbone dans la partie cycloalkyle et 1 à 4 atomes de carbone dans la partie alkyle, et finalement un groupe benzyle ou phényle portant, le cas échéant, un ou plusieurs substituants identiques ou différents, et on peut alors mentionner comme substituants un halogène, un groupe cyano, nitro, alkyle et alcoxy ayant chacun 1 à 4 atomes de carbone, phényle ou phénoxy éventuellement substitué par un halogène, halogénalkyle ou halogénalcoxy ayant chacun 1 à 3 atomes d'halogènes identiques ou différents, et

$R^2$ représente un groupe phényle, phénoxy, phénylthio, phénylsulfinyle ou phénylsulfonyle, chacun portant éventuellement un ou plusieurs substituants identiques ou différents, et on peut alors mentionner comme substituants un halogène, un groupe nitro, cyano, en outre, un groupe alkyle, alcoxy ou alkylthio ayant dans chaque cas 1 à 6 atomes de carbone, ainsi qu'un groupe cyclohexyle, en outre, un groupe halogénalkyle, un groupe halogénalkoxy et un groupe halogénalkylthio ayant chacun 1 à 4 atomes de carbone et 1 à 5 atomes d'halogènes identiques ou différents, en outre, un groupe dialkylamino ayant 1 à 4 atomes de carbone dans la partie alkyle, en outre, un groupe alcoxycarbonyle ayant 1 à 4 atomes de carbone dans la partie alkyle, ainsi que finalement un groupe phényle, phénoxy, phénylthio, benzyle,

benzoxy ou benzylthio portant chacun, le cas échéant, un ou plusieurs substituants halogéno et/ou alkyle ayant 1 à 4 atomes de carbone, caractérisé en ce que:

a) on fait réagir des azolycétones de formule (II):

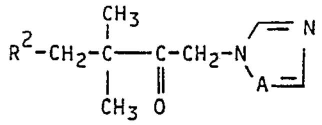 (II)

dans laquelle:

A et $R^2$ ont la définition indiquée ci-dessus,

avec des hydroxylamines substituées de formule (III):

$$H_2N-O-R^1 \qquad (III)$$

dans laquelle:

$R^1$ a la définition indiquée ci-dessus,

le cas échéant, en présence d'un diluant, ou bien

b) on fait réagir les 1-azolyl-2-oximinobutanes de formule générale (Ia) conforme à l'invention:

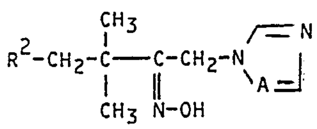 (Ia)

dans laquelle:

A et $R^2$ ont la définition indiquée ci-dessus,

avec des halogènes de formule (IV):

$$Hal-R^1 \qquad (IV)$$

dans laquelle:

$R^1$ a la définition indiquée ci-dessus,

Hal représente du chlore ou du brome,

éventuellement en présence d'une base forte et en présence d'un diluant, ou bien:

c) on fait réagir des 1-halogéno-2-oximinobutanes de formule générale (V):

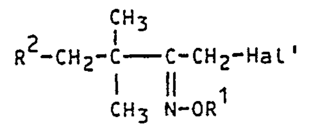 (V)

dans laquelle:

$R^1$ et $R^2$ ont la définition indiquée ci-dessus, et

Hal' représente du chlore ou du brome,

avec le 1,2,4-triazole ou l'imidazole en présence d'un accepteur d'acide et en présence d'un diluant et, le cas échéant, on additionne ensuite, sur les composés de formule (I) ainsi obtenus, un acide ou un sel de métal.

4. Compositions phytosanitaires, caractérisées par une teneur en 1-azolyl-2-oximinobutanes de formule (I) suivant les revendications 1 et 3.

5. Fongicides et compositions influençant la croissance des plantes, caractérisés par une teneur en au moins un 1-azolyl-2-oximinobutane de formule (I) suivant les revendications 1 et 3.

6. Procédé pour combattre des champignons, caractérisé en ce qu'on fait agir un 1-azolyl-2-oximinobutane de formule (I) suivant les revendications 1 et 3 sur des champignons ou sur leur milieu.

7. Procédé pour influencer la croissance de végétaux, caractérisé en ce qu'on fait agir des 1-azolyl-2-oximinobutanes de formule (I) suivant les revendications 1 et 3 sur les plantes et sur leur milieu.

8. Utilisation de 1-azolyl-2-oximinobutanes de formule (I) suivant les revendications 1 et 3 pour combattre des champignons et pour influencer la croissance de végétaux.